# NEUE EUROPÄISCHE PATENTSCHRIFT

(11) **EP 0 870 479 B2**
(45) Veröffentlichungstag und Bekanntmachung des Hinweises auf die Entscheidung über den Einspruch: **05.08.2009**
(45) Hinweis auf die Patenterteilung: 23.11.2005
(21) Anmeldenummer: 98106288.8
(22) Anmeldetag: 06.04.1998
(51) Int. Cl.: A61C 13/08, A61C 13/083, A61C 13/00, A61K 6/06

(54) **Verfahren zur Herstellung eines mehrfarbigen Formkörpers für die Weiterverarbeitung zu einer Zahnrestauration**
Method for the manufactoring of a multi-coloured moulded blank for further processing into a dental restauration
Méthode pour la fabrication d'une ébauche multi-colorée destinée à être façonnée afin d' obtenir une restauration dentaire

(30) Priorität: 07.04.1997 DE 19714178
(43) Veröffentlichungstag der Anmeldung: 14.10.1998
(73) Patentinhaber: Ivoclar Vivadent AG, 9494 Schaan (LI)
(72) Erfinder: Datzmann, Gabriele, 01099 Dresden (DE); Kuhnert, Regina, 01454 Radeberg (DE); Neumann, Michael, 01386 Berlin (DE)
(74) Vertreter: UEXKÜLL & STOLBERG

(56) Entgegenhaltungen:
- EP-A- 0 161 797
- EP-A- 0 272 745
- EP-A- 0 332 887
- WO-A-90//13268
- CH-A- 253 668
- D -A- 2 749 564
- DD-A- 235 557
- DE-A- 1 491 028
- US-A- 599 084
- US-A- 2 677 150
- US-A- 5 342 696
- US-A- 5 653 791
- KARL EICHNER, HEINRICH F. KAPPERT: 'Zahnärztlische Werkstoffe und', Bd. 1, 1996, ISBN 3-7785-2408-9
- HERMANN HEUSCHEL, KLAUS MUCHE: 'ABC Keramik', 31 Mai 1974, VEB DEUTSCHER VERLAG
- FELIX SINGER, SONJA S. SINGER: 'Industrielle Keramik', 1969, SPRINGERVERLAG
- EBERHARD KRAUSE, IRIS BERGER, THEO PLAUL: 'Technologie der Keramik', Bd. 2, VEB VERLAG FÜR BAUWESE

## Beschreibung

Die vorliegende Erfindung betrifft ein Verfahren nach Anspruch 1 zur Herstellung eines mehrfarbigen Formkörpers für die Weiterverarbeitung zu einer Zahnrestauration.

Hintergrund der Erfindung bildet der dentaltechnische Bereich, wobei aus einem keramischen Formkörper oder aus einem Kunststofformkörper die Zahnrestauration, bspw. eine Zahnkrone, ein Inlay, ein Onlay, ein Veneer, herausgearbeitet wird. Das Herausarbeiten erfolgt in zunehmendem Maße maschinell, wobei bspw. das CAD/CAM-Verfahren oder das Kopierfräsverfahren zur Anwendung kommen.

Um den ästhetischen Ansprüchen gerecht zu werden und Zahnrestaurationen mit einer Farbgebung zu erzielen, welche von der Farbe oder auch von den Verfärbungen natürlicher Zähne nicht oder kaum unterscheidbar ist, werden mehrfarbige Formkörper hergestellt.

Aus der EP 0 455 854 A1 ergibt sich bspw. ein keramischer Formkörper aus üblichem keramik- oder Porzellanmaterial mit mehreren farblich unterschiedlichen Schichten von glasartig durchscheinend im okklusalen Bereich bis gelblich opak im zervikalen Bereich. Angaben darüber, wie der mehrschichtige Mehrfarbformkörper hergestellt wird, sind der Druckschrift jedoch nicht zu entnehmen.

Des weiteren offenbart die US 4 970 032 einen mehrfarbigen Kunststofformkörper mit vorgegeben variierenden Farbschichten, die übereinander um einen Kem angeordnet sind. Zur Herstellung des Kerns und der Schichten wird ein Spritzformgebungsverfahren angewendet. Im Ergebnis wird ein Block erhalten, dessen Farbschichten deutlich voneinander abgegrenzt sind, was einer Annäherung an das natürliche Zahnfarbbild nicht zuträglich ist.

Die CH 253668 offenbart Mischungen zur Herstellung keramischer Gegenstände und insbesondere von künstlichen Zähnen, die mindestens zwei Sorten Teilchen aus keramischem Material enthalten, wobei die Teilchen einer Sorte jeweils gleiche aber von den Teilchen der anderen Sorte verschiedene Korngröße und Farbe aufweisen. Die Teilchen werden mit Flüssigkeit angemischt und in Formen gefüllt. Die gefüllte Form wird anschließend einer Vibrationsbehandlung unterworfen, die zu einer Auftrennung der Teilchen entsprechend ihrer Korngröße führen soll. Die Formkörper werden anschließend aus der Form entfernt, getrocknet, gebrannt und glasiert. Ein Verpressen der Ausgangsmaterialien wird nicht erwähnt.

Ausgehend von diesem Stand der Technik liegt die Erfindung die Aufgabe zugrunde, ein Verfahren zur Herstellung eines mehrfarbigen Formkörpers für die Weiterverarbeitung zu einer Zahnrestauration zu schaffen, das für keramische Formkörper anwendbar ist, kostengünstig ist und einen kontinuierlichen Farbverlauf ermöglicht. Die voranstehende Aufgabe wird bezüglich des Verfahrens durch die Merkmale des Patentanspruches 1 gelöst. Danach werden mindestens zwei unterschiedlich gefärbte Ausgangsmaterialien in eine, im wesentlichen die Form des Formkörpers vorgebende Preßmatrize eingefüllt und zum Formkörper verpreßt.

Erfindungsgemäß ist erkannt worden, daß der Farbverlauf optimal und entsprechend den hohen Anforderungen an die Ästhetik des Zahnfarbbildes unabhängig vom Ausgangsmaterial eingestellt werden kann, wenn die Ausgangsmaterialien miteinander verpreßt werden. Die Kontaktflächen werden mit Druck beaufschlagt und die unterschiedlich gefärbten Ausgangsmaterialien werden im Grenzbereich in innigen Kontakt gebracht, so daß dort eine - wenn auch geringe - Vermischung stattfindet. Infolge dieser Vermischung, welche durch die Form und Größe der Teilchen der Ausgangsmaterialien und/oder durch den Preßdruck beeinflußt werden kann, ist ein fließender Farbverlauf erzielbar und der tatsächliche Grenzverlauf zwischen den Ausgangsmaterialien ist nicht ersichtlich. Andererseits können im Wege des Preßverfahrens optional auch deutlich gegeneinander abgegrenzte Farbbereiche erzeugt werden. Durch die Anwendung des Preßverfahrens kann bei niedrigen Kosten infolge geringeren Vorbereitungsaufwandes Keramik als Ausgangsmaterial eingesetzt werden.

Nach einem Ausführungsbeispiel werden die Ausgangsmaterialien sukzessive, bevorzugt schichtförmig, in die Preßmatritze eingefüllt. Dabei können mehrere Schichten gebildet werden, die unterschiedliche oder gleiche Schichtdicken aufweisen. Die dabei gebildeten Schichten können horizontal und/oder vertikal verlaufen, wobei insbesondere für die Bildung von vertikalen Schichten eine Trennwand vorgesehen sein kann, die vor dem Verpressen herausgenommen wird. Alternativ könnte auch eine Befüllung derart erfolgen, daß gleichzeitig mehrere Ausgangsmaterialien an verschiedenen Punkten der Matritze eingefüllt werden, wobei sich ein vertikaler Schichtverlauf ergeben kann. Die Ausgangsmaterialien könnten bspw. auch ringförmig verfüllt werden, was bspw. bei Kronen von Vorteil ist, wobei es auf die Lage der Farbanteile besonders ankommt.

Der Formkörper wird im Wege des Trockenpressens erstellt, wobei die Teilchen der Ausgangsmaterialien an den Grenzflächen besonders innig ineinandergreifen und die Mischungsschicht ausbilden. In bekannter Weise könnten die Ausgangsmaterialen zum Trockenpressen Preßhilfsmittel, wie Gleitmittel oder Binder, zugesetzt werden. Für keramische Ausgangsmaterialen könnten als Binder Polyvinylalkkohole, Cellulosederivate und Alginate und als Gleitmittel Wachs verwendet werden.

Gemäß einem weiteren Ausführungsbeispiel könnten die Ausgangsmaterialien während des Trockenpressens mit Temperatur beaufschlagt werden. Hierdurch werden auch andere Eigenschaften als die Farbigkeit beeinflußt, insbesondere die Festigkeit des Formkörpers im einerseits und im Bereich der aneinandergrenzenden Ausgangsmaterialien andererseits. Bei keramischen Ausgangsmaterialien kommt die Heißisostatik, bei Kunststoffen das Heißpressen mit erhitzten Werkzeugen in Betracht.

Der verpreßte und aus der Matrize entfernte Formkörper könnte außerdem einer Temperaturbehandlung unterzogen werden, insbesondere um die Festigkeit zu erhöhen. Auch andere Gefügeeigenschaften können bei einer nachgeschalteten Temperaturbehandlung eingestellt werden.

Die bereits voranstehend erläuterte vorteilhafte Möglichkeit, durch das Preßverfahren kontinuierlich ineinander übergehende Ausgangsmaterialien bzw. Schichten zu erzeugen, könnte durch den Einsatz stärker oder schwächer kontrastierender Farben auf Wunsch manipuliert werden.

Damit eine gute Kompatibilität der Ausgangsmaterialien untereinander gewährleistet ist, könnten diese im wesentlichen dieselbe chemische Zusammensetzung aufweisen und sich nur im Hinblick auf die Färbung unterscheiden. Was die physikalische Beschaffenheit der Ausgangsmaterialien angeht, so können diese als Pulver, Granulat oder plastische Masse vorliegen. Auch hier ist es von Vorteil, wenn alle Ausgangsmaterialien für einen Formkörper in der selben verpreßbaren Form eingesetzt werden.

Wie bereits oben erwähnt, wird als Ausgangsmaterial ein keramischer Werkstoff verwendet. Es wird ein keramischer Werkstoff eingesetzt, der synthetisch hergestellt ist und auf Metalloxiden, nämlich zumindest SiO₂, K₂O und Al₂O₃, und/oder deren Nitraten und Carbonaten basiert. Der keramische Werkstoff könnte auch aus Mischungen von Oxidkeramik und dem synthetisierten Werkstoff bestehen.

Im Vergleich zu keramischen Formkörpern aus natürlicher Feldspatkeramik ist die synthetische Keramik sehr vorteilhaft. Bei der natürlichen Feldspatkeramik werden natürliche Rohstoffe eingesetzt, was mit einigen Nachteilen verbunden ist. Es können nur sehr reine Feldspäte verwendet werden, um den optischen Ansprüchen von Zahnrestaurationen zu genügen. Die Beschaffung derart reiner Rohstoffe ist schwierig und kostspielig. Selbst sehr reine Feldspäte müssen handverlesen werden, da natürlich vorkommende Verunreinigungen selektiert werden müssen. Außerdem weisen die natürlichen Feldspäte in Abhängigkeit von der geologischen Vorgeschichte unterschiedliches Schmelzverhalten auf, was eine Reproduzierbarkeit des Hersteltungsverfahrens - bspw. im Hinblick auf Brenntemperaturen und Brennzeiten - erschwert und die Qualität beeinträchtigt.

Bezüglich der Verwendung einer synthetischen Keramik ist als vorteilhaft erkannt worden, daß eine kostengünstige Herstellung, eine im wesentlichen schwankungsfreie Qualität und damit auch eine gute Reproduzierbarkeit eines Formkörpers realisiert werden kann, wenn dieser aus einem synthetischen Werkstoff besteht und wenn bei dem Verfahren als Ausgangsmaterialien reine Metalloxide oder deren Nitrate oder Carbonate oder Metalloxidverbindungen eingesetzt werden. Auf diese Weise werden naturgegebene Verunreinigungen oder Beschaffungsprobleme natürlicher Rohstoffe ausgeschaltet. Zudem ergeben sich die Kostenvorteile auch aus der großtechnischen Herstellbarkeit der reinen Metalloxide oder - verbindungen, deren gleichbleibender Produktqualität, kürzeren Fertigungszeiten bei der Verarbeitung und einer höheren Transparenz aufgrund der Reinheit.

Der bevorzugte synthetische keramische Werkstoff, welcher - ähnlich wie bei einem Baukastensystem - mit weiteren Stoffen zur Erzielung bestimmter Eigenschaften modifiziert werden kann, weist folgenden Grundversatz auf:

| | | | | |
|---|---|---|---|---|
| SiO₂ | 58 | bis | 65 | % |
| Al₂O₃ | 10 | bis | 16 | % |
| K₂O | 12 | bis | 18 | % |
| Na₂O | 1 | bis | 5 | % |
| CaO | 1 | bis | 5 | % |
| Li₂O | 0 | bis | 5 | % |
| B₂O₃ | 0 | bis | 5 | % |

Der synthetische keramische Werkstoff wird dadurch gewonnen wird, daß die Metalloxide, deren Karbonate und/oder Nitrate vorzugsweise trocken gemischt werden, daß das Gemisch zum Erhalt einer leucithaltigen Fritte vorzugsweise bei Temperaturen von 1350°C bis 1600°C geschmolzen oder gesintert wird und die so erhaltene Fritte bei Temperaturen von 600°C bis 1000°C getempert und anschließend in eine verpreßbare Form verbracht wird.

Während des Schmelzens oder Sinterns des Metalloxidgemisches werden zwei Phasen - nämlich die Leucitkristalle einerseits und die Glasphase andererseits - ausgebildet. Zur gezielten qualitativen und quantitativen Einstellung der Leucitkristalle, könnte sich ein gezielter Tempervorgang mit Haltezeiten in verschiedenen Temperaturbereichen von ca. 1 h bis 10 h anschließen. Damit eine Weiterverarbeitung der Fritte zum Formkörper erfolgen kann, wird diese in eine für das Preßverfahren geeignete Form überführt. Zunächst wird die in großen Stücken vorliegende getemperte Fritte auf eine Korngröße von ca. 10 µm zerkleinert. Das so erhaltene Pulver könnte nun direkt zum Formkörper verpreßt werden. Im Hinblick auf eine bessere Verarbeitbarkeit wird es jedoch bevorzugt, das Pulver zu dispergieren, in einen Sprüh- oder Wirbelschichttrockner zu pumpen und ein Granulat zu gewinnen, welches zum Formkörper verpreßt wird.

Bevor die Formgebung des Formkörpers stattfindet, könnte die Fritte mit weiteren Stoffen modifiziert werden, so daß gezielt Eigenschaften des Ausgangsmaterials eingestellt werden können. Die Modifizierung der Fritte erfolgt bevorzugt nach dem Zerkleinern. Der weitere Stoff könnte mit dem Frittenpulver bspw. in einer Mühle trocken vermischt werden oder auch zu einem Schlicker dispergiert werden. Der Herstellung eines Schlickers wird dann der Vorzug gegeben, wenn ohnehin ein Granulat zum Pressen bereitgestellt werden soll.

Mit der Modifizierung der Fritte könnten bspw. der Wärmeausdehnungskoeffizient, die Brenntemperatur, die Transparenz oder die Festigkeit verändert werden. Zur gezielten Beeeinflussung der vorgenannten Eigenschaften könnte die Fritte mit einer weiteren Fritte verschnitten werden.

Zur Erhöhung der Festigkeit und/oder des Wärmeausdehnungskoeffizienten hat sich zwei Fritten mit folgenden Grundversätzen herausgestellt:

| 1.) | | | | |
|---|---|---|---|---|
| SiO₂ | 50 | bis | 55 | % |
| Al₂O₃ | 18 | bis | 25 | % |
| K₂O | 13 | bis | 20 | % |
| Na₂O | 1 | bis | 5 | % |
| CaO | 0 | bis | 3 | % |
| Li₂O | 0 | bis | 5 | % |
| TiO₂ | 2 | bis | 8 | % |

| 2.) | | | | |
|---|---|---|---|---|
| SiO₂ | 50 | bis | 55 | % |
| Al₂O₃ | 18 | bis | 25 | % |
| K₂O | 13 | bis | 20 | % |
| Na₂O | 1 | bis | 5 | % |
| CaO | 0 | bis | 3 | % |
| Li₂O | 0 | bis | 5 | % |
| TiO₂ | 0 | bis | 3 | % |
| B₂O₃ | 0 | bis | 3 | % |

Die Erhöhung der Festigkeit spielt im Hinblick auf die Kaubelastungen der Zahnrestauration eine große Rolle. Des weiteren ist die hohe Festigkeit auch für die Weiterverarbeitung des Formkörpers zur Zahnrestauration relevant. Bei der Weiterverarbeitung kommen diverse Trennwerkzeuge, wie Fräs- und Schleifwerkzeuge, zum Einsatz, welche den Formkörper mit großen Kräften beaufschlagen.

Zur Verbesserung der Transparenz hat sich eine Fritte mit nachfolgend aufgeführtem Grundversatz als vorteilhaft erwiesen:

| | | | | |
|---|---|---|---|---|
| SiO₂ | 60 | bis | 65 | % |
| Al₂O₃ | 10 | bis | 15 | % |
| K₂O | 10 | bis | 15 | % |
| Na₂O | 2 | bis | 7 | % |
| CaO | 1 | bis | 5 | % |
| Li₂O | 0 | bis | 1 | % |
| TiO₂ | 0 | bis | 3 | % |
| B₂O₃ | 0 | bis | 3 | % |

Auch die Luminescenz der Fritte könnte beeinflußt werden, was gerade mit Blick auf die Anwendung der Zahnrestauration und die damit verbundenen ästhetischen Ansprüche von Bedeutung ist. Insbesondere könnte die Fritte zur Einstellung der Fluoreszenz mit einem Fluoreszenzmittel versetzt werden.

Eine weitere und für die erfindungsgemäße Lehre im Hinblick auf die Mehrfarbigkeit der Formkörper wesentliche Modifizierung der Fritte stellt deren farbliche Einstellung dar. Im zahntechnischen Bereich müssen unterschiedlichste Zahnfarben bereitgestellt werden, um dem individuellen Zahnfarbbild der Patienten entsprechen zu können. Der Fritte könnten verschiedene Pigmente zugesetzt werden, so daß eine Sortiment unterschiedlich gefärbter Fritten bereitgestellt werden kann und allerfeinste Farbabstufungen, wie sie am einzelnen natürlichen Zahn vorkommen, realisierbar sind.

Gemäß einem weiteren Ausführungsbeispiel könnte eine Schicht eines temperaturbehandelten, mehrfarbigen Formkörpers folgenden Grundversatz aufweisen:

| | | | | |
|---|---|---|---|---|
| SiO₂ | 58 | bis | 65 | % |
| Al₂O₃ | 13 | bis | 20 | % |
| K₂O | 11 | bis | 18 | % |
| Na₂O | 1 | bis | 6 | % |
| CaO | 0 | bis | 4 | % |
| Li₂O | 0 | bis | 1 | % |
| TiO₂ | 0 | bis | 2 | % |
| B₂O₃ | 0 | bis | 4 | % |

An dieser Stelle wird ausdrücklich darauf hingewiesen, daß die Grundversätze und die modifizierten Grundversätze des synthetischen keramischen Werkstoffes sehr wohl auch zur Herstellung einfarbiger keramischer Formkörper eingesetzt werden können.

Der infolge der Preßformgebung gewonnene Formkörper wird nun bei Temperaturen von 700°C bis 1200°C und zwar vorzugsweise unter Vacuum gesintert. Die Sinterung unter Vacuum wirkt sich positiv auf die Transparenz aus, da Gaseinschlüsse vermieden werden. Als besonders vorteilhaft hat sich eine anschließende Temperung des Formkörpers erwiesen, wobei der Leucitanteil und die Größe der Leucitkristalle eingestellt wird. Um eine gleichmäßige Temperaturverteilung am und im Formkörper zu erzielen, wird ein Formkörper mit horizontaler Farbschichtung stehend gebrannt, so daß alle Farbschichten an der Stirnseite des Formkörpers die Brennunterlage kontaktieren.

Der temperaturbehandelte Formkörper weist eine Festigkeit von mehr als 100 MPa auf, bevorzugt eine Festigkeit von 100 bis 300 MPa, stärker bevorzugt von 100 bis 180 MPa.

Hervorzuheben ist die kostengünstige Herstellung, da es sich um eine synthetische Glaskeramik handelt, die auf synthetisch gewonnenen und damit großtechnisch und kostengünstig herstellbaren Ausgangsstoffen basiert. Außerdem weist ein solcher Formkörper eine hohe Transparenz und einen sog. Chamäleoneffekt auf, der eine gute Anpassung an die natürliche Zahnfarbe ermöglicht. Des weiteren ist der Zweiphasenaufbau der Glaskeramik - nämlich Leucit und Glas - durch die sehr gute Ätzbarkeit einer qualitativ guten und funktionssicheren adhäsiven Befestigung am Zahn förderlich.

Unter dem Aspekt der maschinellen Weiterverarbeitung des keramischen Formkörpers zu einer Zahnrestauration, nämlich Inlays, Onlays, Veneers oder Kronen, ist es von besonderem Vorteil, wenn der Formkörper mit einer Halterung konfektioniert werden kann. Die Halterung könnte dann in bekannter Weise in einer Bearbeitungsmaschine integriert werden. Denkbar ist es ebenso, den Formkörper direkt und ohne Halterung in eine Bearbeitungsmaschine einzuspannen.

Zur Durchführung des erfindungsgemäßen Verfahrens sieht die Erfindung eine Vorrichtung vor, bei der einer Presse, insbesondere einer Trockenpresse, separate Hilfseinrichtungen, wie Rutschen, Schütten, Rohrzuführungen, Vibrierrinnen, Füllschuhe, zum getrennten Befüllen der Matritze des Preßwerkzeuges mit den unterschiedlich gefärbten Ausgangsmaterialien zuordenbar sind.

Es haben sich Füllschuhe bewährt zum sukzessiven Befüllen der Matrize des Preßwerkzeuges mit den unterschiedlich gefärbten Ausgangsmaterialien. Je nach dem, welche Farbschicht erzeugt werden soll und je nach Höhe der Befüllung wird der jeweilige Füllschuh an der Trockenpreßmaschine angeordnet und in die Matrize ausgeleert. Von besonderem Vorteil ist es, wenn für jede Farbnuance der unterschiedlich gefärbten Ausgangsmaterialien ein Füllschuh vorgesehen ist. Auf diese Weise wird Verunreinigungen und damit verbundenen Farbabweichungen vorgebeugt.

Über das Preßwerkzeug lassen sich die unterschiedlichsten Formen des Formkörpers realisieren. Nach einem Ausführungsbeispiel könnte das Preßwerkzeug derart ausgestaltet sein, daß beim Pressen des Formkörper eine Vertiefung ausgebildet wird. Die Vertiefung ist im Hinblick auf die Innenbearbeitung einer Zahnkrone vorteilhaft. Gemäß einer weiteren Ausführungsform könnte der keramische Formkörper bereits im Formgebungsverfahren mit einer Vertiefung für die Innenbearbeitung einer Zahnkrone versehen werden. Hierzu ist es notwendig, daß das Preßwerkzeug zur Erstellung des Vertiefung eine entsprechende Ausgestaltung aufweist.

Zur Verteilung der Ausgangsmaterialien in der Matrize könnte eine herausnehmbare Trennwand vorgesehen sein, die in die Matrize eingesetzt wird und unmittelbar vor dem Pressen entnommen wird.

Die Erfindung kann auch vorsehen, daß der Preßstempel, der auf die eingefüllten Materialien einwirkt, formgebend ausgebildet ist.

## Patentansprüche

1. Verfahren zur Herstellung eines mehrfarbigen Formkörpers für die Weiterverarbeitung zu einer Zähnrestauration, welches die folgenden Schritte aufweist:
a) Befüllen einer Preßmatrize, welche im wesentlichen die Form des Formkörpers vorgibt, mit mindestens zwei unterschiedlich gefärbten keramischen Werkstoffen, die eine Granulatform haben,
b) Verpressen der in die Preßmatrize eingefüllten keramischen Werkstoffe zu dem Formkörper
c) Sintern des Formkörperrohlings bei Temperaturen von 700 °C bis 1200 °C,
wobei der keramische Werkstoff ein synthetischer Werkstoff ist, welcher auf SiO₂, K₂O und Al₂O₃ basiert.

2. Verfahren nach Anspruch 1, **dadurch gekennzeichnet, daß** zur Herstellung des granulatförmigen keramischen Werkstoffs einem Basismaterial vor dem Granulieren ein oder mehrere Pigmente und/oder Fluoreszenzmittel zugesetzt werden.

3. Verfahren nach Anspruch 1 oder 2, **dadurch gekennzeichnet, daß** der synthetische keramische Werkstoff folgenden Grundversatz aufweist:
| | |
|---|---|
| SiO₂ | 58 bis 65 % |
| Al₂O₃ | 10 bis 16 % |
| K₂O | 12 bis 18 % |
| Na₂O | 1 bis 5 % |
| CaO | 1 bis 5 % |
| Li₂O | 0 bis 5 % |
| B₂O₃ | 0 bis 5 % |

4. Verfahren nach einem der Ansprüche 1 bis 3, **dadurch gekennzeichnet, daß** der synthetische keramische Werkstoff **dadurch** gewonnen wird, daß die Metalloxide, deren Karbonate und/oder Nitrate vorzugsweise trocken gemischt werden, daß das Gemisch zum Erhalt einer leucithaltigen Fritte vorzugsweise bei Temperaturen von 1350 °C bis 1600 °C geschmolzen oder gesintert wird und die so erhaltene Fritte bei Temperaturen von 600 °C bis 1000 °C getempert und anschließend in eine verpreßte Form verbracht wird.

5. Verfahren nach Anspruch 4, **dadurch gekennzeichnet, daß** die Fritte zur Erhöhung der Festigkeit und /öder des Wärmeausdehnungskoeffizienten mit einer weiteren Fritte verschnitten wird, die folgenden Grundversatz aufweist:
| | |
|---|---|
| SiO₂ | 50 bis 55 % |
| Al₂O₃ | 18 bis 25 % |
| K₂O | 13 bis 20 % |
| Na₂O | 1 bis 5 % |
| CaO | 0 bis 3 % |
| Li₂O | 0 bis 5 % |
| TiO₂ | 2 bis 8 % |

6. Verfahren nach Anspruch 4 oder 5, **dadurch gekennzeichnet, daß** die Fritte zur Erhöhung der Festigkeit und/oder des Wärmeausdehnungskoeffizienten mit einer weiteren Fritte veschnitten wird, die folgenden Grundversatz aufweist:
| | |
|---|---|
| SiO₂ | 50 bis 55 % |
| Al₂O₃ | 18 bis 25 % |
| K₂O | 13 bis 20 % |
| Na₂O | 1 bis 5 % |
| CaO | 0 bis 3 % |
| Li₂O | 0 bis 5 % |
| TiO₂ | 0 bis 3 % |
| B₂O₃ | 0 bis 3 % |

7. Verfahren nach einem der Ansprüche 4 bis 6, **dadurch gekennzeichnet, daß** die Fritte zur Verbesserung der Transparenz mit einer weiteren Fritte verschnitten wird, die folgenden Grundversatz aufweist:
| | |
|---|---|
| SiO₂ | 60 bis 65 % |
| Al₂O₃ | 10 bis 15 % |
| K₂O | 10 bis 15 % |
| Na₂O | 2 bis 7 % |
| CaO | 1 bis 5 % |
| Li₂O | 0 bis 1 % |
| TiO₂ | 0 bis 3 % |
| B₂O₃ | 0 bis 3 % |

8. Verfahren nach einem der Ansprüche 1 bis 7, **dadurch gekennzeichnet, daß** der synthetische keramische Werkstoff eines Formkörpers folgenden Grundversatz aufweist:
| | |
|---|---|
| SiO₂ | 58 bis 65 % |
| Al₂O₃ | 13 bis 20 % |
| K₂O | 11 bis 18 % |
| Na₂O | 1 bis 6 % |
| CaO | 0 bis 4 % |
| Li₂O | 0 bis 1 % |
| TiO₂ | 0 bis 2 % |
| B₂O₃ | 0 bis 4 % |

9. Verfahren nach einem der Ansprüche 1 bis 8, **dadurch gekennzeichnet, daß** der Formkörperrohling unter Vakuum gesintert wird.

10. Verfahren nach einem der Ansprüche 1 bis 9, **dadurch gekennzeichnet, daß** der Formkörper einem Tempervorgang unterworfen wird.

11. Verfahren nach einem der Ansprüche 1 bis 10, **dadurch gekennzeichnet, daß** die keramischen Werkstoffe schichtförmig in die Preßmatritze eingefüllt werden.

12. Verfahren nach einem der Ansprüche 1 bis 11, **gekennzeichnet durch** den Schritt des Trockenpressens der keramischen Werkstoffe.

13. Verfahren nach Anspruch 12, **dadurch gekennzeichnet, daß** die keramischen Werkstoffe während des Trockenpressens mit Temperatur beaufschlagt werden.

14. Verfahren zur Herstellung einer Zahnrestauration, **dadurch gekennzeichnet, daß** man gemäß einem der Verfahren nach einem der Ansprüche 1 bis 13 einen Formkörper herstellt und mit einer Halterung konfektioniert und maschinell zu einer Zahnrestauration weiterverarbeitet.

## Claims

1. A method for producing a multi-coloured, shaped body suitable for further processing into a dental restoration comprising the following steps:
a) filling a compression die, which essentially predetermines the shape of the moulded body, with at least two differently coloured ceramic materials that are granular in form,
b) compressing the ceramic materials introduced into the compression die to the moulded body.
c) sintering the moulded body blank at temperatures of 700 °C to 1200°C,
wherein the ceramic material is a synthetic material that is based on SiO₂, K₂O and Al₂O₃.

2. Process according to Claim 1, **characterised in that** in order to produce the granular ceramic material, one or a plurality of pigments and/or fluorescence agents are added to a base material prior to granulation.

3. Process according to Claim 1 or 2, **characterised in that** the synthetic ceramic material has the following basic composition:
| | |
|---|---|
| SiO₂ | 58 to 65% |
| Al₂O₃ | 10 to 16% |
| K₂O | 12 to 18% |
| Na₂O | 1 to 5% |
| CaO | 1 to 5% |
| Li₂O | 0 to 5% |
| B₂O₃ | 0 to 5% |

4. Process according to one of Claims 1 to 3, **characterised in that** the synthetic ceramic material is obtained by mixing the preferably dry metal oxides, the carbonates and/or nitrates thereof, that the mixture is preferably melted or sintered at temperatures of 1350 °C to 1600 °C to obtain a leucite-containing frit, and the resulting frit is tempered at temperatures of 600°C to 1000°C and subsequently brought into a compressed shape.

5. Process according to Claim 4, **characterised in that** the frit, in order to increase the strength and/or the coefficient of thermal expansion, is blended with a further frit, which has the following basic composition:
| | |
|---|---|
| SiO₂ | 50 to 55% |
| Al₂O₃ | 18 to 25% |
| K₂O | 13 to 20% |
| Na₂O | 1 to 5% |
| CaO | 0 to 3% |
| Li₂O | 0 to 5% |
| TiO₂ | 2 to 8% |

6. Process according to Claim 4 or 5, **characterised in that** the frit, in order to increase the strength and/or the coefficient of thermal expansion, is blended with a further frit, which has the following basic composition:
| | |
|---|---|
| SiO₂ | 50 to 55% |
| Al₂O₃ | 18 to 25% |
| K₂O | 13 to 20% |
| Na₂O | 1 to 5% |
| CaO | 0 to 3% |
| Li₂O | 0 to 5% |
| TiO₂ | 0 to 3% |
| B₂O₃ | 0 to 3% |

7. Process according to one of Claims 4 to 6, **characterised in that** the frit, in order to increase the transparency, is blended with a further frit, which has the following basic composition:
| | |
|---|---|
| SiO₂ | 60 to 65% |
| Al₂O₃ | 10 to 15% |
| K₂O | 10 to 15% |
| Na₂O | 2 to 7% |
| CaO | 1 to 5% |
| Li₂O | 0 to 1% |
| TiO₂ | 0 to 3% |
| B₂O₃ | 0 to 3% |

8. Process according to one of Claims 1 to 7, **characterised in that** the synthetic ceramic material of a moulded body has the following basic composition:
| | |
|---|---|
| SiO₂ | 58 to 65% |
| Al₂O₃ | 13 to 20% |
| K₂O | 11 to 18% |
| Na₂O | 1 to 6% |
| CaO | 0 to 4% |
| Li₂O | 0 to 1% |
| TiO₂ | 0 to 2% |
| B₂O₃ | 0 to 4% |

9. Process according to one of Claims 1 to 8, **characterised in that** the moulded body blank is sintered under vacuum.

10. Process according to one of Claims 1 to 9, **characterised in that** the moulded body is subjected to a tempering step.

11. Process according to one of Claims 1 to 10, **characterised in that** the ceramic materials are introduced into the compression die in layer form.

12. Process according to one of Claims 1 to 11, **characterised by** the step of dry-pressing the ceramic materials.

13. Process according to one of Claim 12, **characterised in that** the ceramic materials are subjected to the action of heat during the dry-pressing operation.

14. Process for manufacturing a dental restoration, **characterised in that** a moulded body is manufactured according to one of the processes of one of the Claims 1 to 13 and is assembled with a retaining device and then further machine-processed to form a dental restoration.

## Revendications

1. Procédé de fabrication d'un corps moulé à couleurs multiples destiné à être façonné afin d'obtenir une restauration dentaire, comprenant les étapes suivantes :
a) remplissage d'une matrice de compression, qui prédétermine sensiblement la forme du corps moulé, avec au moins deux matériaux céramiques différemment colorés qui se présentent sous une forme granulaire;
b) compression des matériaux céramiques introduits dans la matrice de compression pour obtenir le corps moulé, et
c) frittage de l'ébauche de corps moulé à des températures de 700 °C à 1200 °C,
dans lequel le matériau céramique est un matériau de synthèse à base de SiO₂, de K₂O et d'Al₂O₃.

2. Procédé selon la revendication 1, **caractérisé en ce que**, pour fabriquer le matériau céramique granulaire, on ajoute à un matériau de base, avant granulation, un ou plusieurs pigments et/ou agents fluorescents.

3. Procédé selon la revendication 1 ou 2, **caractérisé en ce que** le matériau céramique de synthèse présente la composition de base suivante :
| | |
|---|---|
| SiO₂ | 58 à 65 % |
| Al₂O₃ | 10 à 16 % |
| K₂O | 12 à 18% |
| Na₂O | 1 à 5 % |
| CaO | 1 à 5 % |
| Li₂O | 0 à 5 % |
| B₂O₃ | 0 à 5 %. |

4. Procédé selon l'une quelconque des revendications 1 à 3, **caractérisé en ce que** le matériau céramique de synthèse est obtenu en mélangeant les oxydes métalliques, leurs carbonates et/ou leurs nitrates de préférence à sec, **en ce que** le mélange est fondu ou fritté pour obtenir une fritte contenant de la leucite, de préférence à des températures de 1350 °C à 1600 °C, et **en ce que** la fritte ainsi obtenue est soumise à une trempe à des températures de 600 °C à 1000 °C et ensuite transférée dans un moule comprimé.

5. Procédé selon la revendication 4, **caractérisé en ce que**, pour augmenter la résistance et/ou le coefficient de dilatation thermique, la fritte est diluée avec une autre fritte présentant la composition de base suivante :
| | |
|---|---|
| SiO₂ | 50 à 55 % |
| Al₂O₃ | 18 à 25 % |
| K₂O | 13 à 20 % |
| Na₂O | 1 à 5 % |
| CaO | 0 à 3 % |
| Li₂O | 0 à 5 % |
| TiO₂ | 2 à 8 %. |

6. Procédé selon la revendication 4 ou la revendication 5, **caractérisé en ce que**, pour augmenter la résistance et/ou le coefficient de dilatation thermique, la fritte est diluée avec une autre fritte présentant la composition de base suivante :
| | |
|---|---|
| SiO₂ | 50 à 55 % |
| Al₂O₃ | 18 à 25 % |
| K₂O | 13 à 20 % |
| Na₂O | 1 à 5 % |
| CaO | 0 à 3 % |
| Li₂O | 0 à 5 % |
| TiO₂ | 0 à 3 % |
| B₂O₃ | 0 à 3 %. |

7. Procédé selon l'une quelconque des revendications 4 à 6, **caractérisé en ce que**, pour améliorer la transparence, la fritte est diluée avec une autre fritte présentant la composition de base suivante :
| | |
|---|---|
| SiO₂ | 60 à 65 % |
| Al₂O₃ | 10 à 15 % |
| K₂O | 10 à 15 % |
| Na₂O | 2 à 7% |
| CaO | 1 à 5 % |
| Li₂O | 0 à 1 % |
| TiO₂ | 0 à 3 % |
| B₂O₃ | 0 à 3 %. |

8. Procédé selon l'une quelconque des revendications 1 à 7, **caractérisé en ce que** le matériau céramique de synthèse d'un corps moulé présente la composition de base suivante :
| | |
|---|---|
| SiO₂ | 58 à 65 % |
| Al₂O₃ | 13 à 20 % |
| K₂O | 11 à 18 % |
| Na₂O | 1 à 6 % |
| CaO | 0 à 4 % |
| Li₂O | 0 à 1 % |
| TiO₂ | 0 à 2 % |
| B₂O₃ | 0 à 4 %. |

9. Procédé selon l'une quelconque des revendications 1 à 8, **caractérisé en ce que** l'ébauche de corps moulé est frittée sous vide.

10. Procédé selon l'une quelconque des revendications 1 à 9, **caractérisé en ce que** le corps moulé est soumis à une opération de trempe.

11. Procédé selon l'une quelconque des revendications 1 à 10, **caractérisé en ce que** les matériaux céramiques sont introduits sous forme de couches dans la matrice de compression.

12. Procédé selon l'une quelconque des revendications 1 à 11, **caractérisé par** l'étape de compression à sec des matériaux céramiques.

13. Procédé selon la revendication 12, **caractérisé en ce qu'**une température est appliquée aux matériaux céramiques pendant la compression à sec.

14. Procédé de fabrication d'une restauration dentaire, **caractérisé en ce que** l'on fabrique un corps moulé selon l'un des procédés de l'une quelconque des revendications 1 à 13, on le moule avec une fixation et on le travaille à la machine pour obtenir une restauration dentaire.
